# EUROPEAN PATENT SPECIFICATION

(11) **EP 1 200 088 B1**
(45) Date of publication and mention of the grant of the patent: **04.06.2003**
(21) Application number: 00949829.6
(22) Date of filing: 28.07.2000
(51) Int. Cl.: A61K 31/47

(54) **PARACETAMOL AND DROTAVERINE CONTAINING COMPOSITION**
PARACETAMOL UND DROTAVERIN ENTHALTENDE ZUSAMMENSETZUNG
COMPOSITION PHARMACEUTIQUE CONTENANT PARACETAMOL ET DROTAVERINE

(30) Priority: 28.07.1999 HU 9902559
(43) Date of publication of application: 02.05.2002
(73) Proprietor: CHINOIN Gyògyszer és Vegyészeti Termékek Gyára RT., 1045 Budapest IV (HU)
(72) Inventor: VITANYINE MORVAI, Magdolna, H-1054 Budapest (HU); KOVACS, Péterné, H-1025 Budapest (HU); KALMANNE MATHE, Irma, H-2083 Solymár (HU); JAKAB, Boglárka, H-1063 Budapest (HU); VEGELI, Erzsébet, H-1131 Budapest (HU)
(74) Representative: Schwarz, Albin, Dr.
(86) International application number: HU0000086
(87) International publication number: WO01007024

(56) References cited:
- US-A- 5 073 379
- US-A- 5 348 745
- WOJCICKI, JERZY ET AL: "Effect of papaverine and atropine on pharmacokinetics of paracetamol administered orally" POL. J. PHARMACOL. PHARM. (1979), 31(3), 239-43 , XP000974559

## Description

The subject of the present invention is a new analgetic / antipyretic composition in tablet form containing as active ingredient paracetamol and drotaverine hydrochloride or paracetamol, drotaverine hydrochloride and codein in combination. The new combination can be applied orally, is stable in physicochemical sense too, and absorbs quickly. The invention relates also to the preparation of the new composition.

It is known that a number of pharmaceutical preparations with different chemical structures and the combinations of them are applied for treating diseases accompanied with pains of different origin, e.g. headache or febrile, pyretic conditions (e.g. paracetamol, novaminophenazone, phenazone, acetylsalicylic acid and their combinations, namely paracetamol + codein, paracetamol + caffein or novaminophenazone + caffein are used for reducing fever and/or pain). The combinations facilitate reduction of doses of the single active ingredients, holding so their harmful side effects, and reduce the pains effectively in a short time.

One of the active ingredients of the composition of the present invention is paracetamol (p-acetylamino phenol), possessing of powerful antipyretic and analgetic effect.

The other active ingredient of the composition of the present invention is drotaverine HCl, a compound containing isoquinoline skeleton (3,4-dihydroperparine HCl salt), the original molecule of Chinoin Pharmaceutical and Chemical Works Co. Ltd., Budapest., used in the therapy as spasmolytic under the trade name of NO-SPA. It is used in itself or in combination with an analgetic (e.g. novaminophenazone) agent.

An additional active ingredient used in the composition of the present invention is codein, the 3-methylether of morphine. It is absorbed orally well, suitable to terminate medium strong pains, a frequent component of non narcotic analgetics.

The chemical stability of the single active ingredients used in the combination of the present invention is different.

According to literature data paracetamol is incompatible with strong acids, because acidic conditions facilitate the hydrolysis of it, which results in the production of a toxic by-product, the p-aminophenol.
To decrease the side effect of paracetamol numerous compositions have been developed, which contain beside paracetamol caffein, acid binding auxiliary material(s), citric acid and ascorbic acid (Vitamin C). In these compositions the advantageous property of paracetamol in comparisson with aspirin is, that it does not damage the mucosa of the stomach.

The mixture of paracetamol, acetylsalicylic acid and caffein is widely used for relief of pain. In the above composition polyvinyl-butyrate is used for securing the chemical stability of acetylsalicylic acid and hydroxypropyl cellulose to facilitate the disintegration.

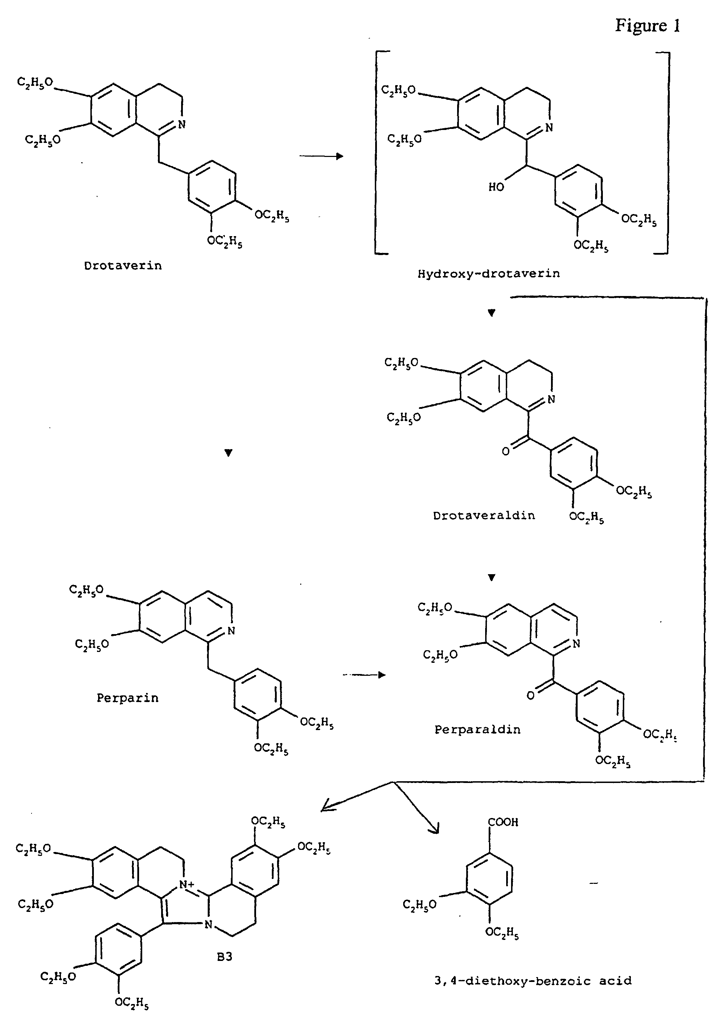

In neutral and alkaline medium the drotaverine HCI oxidizes to drotaveraldine by an oxidative degradation mechanism, while upon the effect of light it decomposes to perparine and finally to perparaldine by dehydrogenation of the molecule (Figure 1). Both types of decomposition can be inhibited by applying an antioxidant or an acidic auxiliary material.
During the compatibility tests we have found that the chemical stability of solid state pharmaceutical preparations containing drotaverine hydrochloride was significantly influenced by the tabletting vehicles, by the moisture content of the granules and the tablets, furthermore by the pH-conditions of the formula.

We have studied the interaction of drotaverine HCl and numerous tabletting vehicles, and found that the decomposition of drotaverine HCI is significantly enhanced by magnesium stearate, the glider vehicle widely used during tabletting, which'hydrolyzes in an alkaline way.

Codein phosphate does not exhibit significant decomposition in oral, solid pharmaceutical preparations. According to literature data by reacting codein phosphate in solid state with organic acids, e.g. citric acid or the most frequently used gliders, magnesium stearate or stearic acid, it is able to transesterification (J. of Pharm. Sci., Vol.76. No.1., January, 1987).

A patent specification of Mallinckrodt Inc. (EP 0 040 472 A2) describes the preparation of a combination, containing 300-650 mg paracetamol and 15-60 mg codein phosphate. During the preparation spray-drying manufacturing technology and 1.5 % to 25 % pretreated starch as vehicle were used. Paracetamol (100 - 600 mg) and codein phosphate (5 - 60 mg) are also favourably used in muscle relaxant compositions.

US 5073379 describes a process for the preparation of solid pharmaceutical forms containg active ingredients such as paracetamol and drotaverine.

The goal of our invention was to produce oral pharmaceutical compositions containing paracetamol and drotaverine HCl, or paracetamol. drotaverine HCl and codein phosphate, with appropriate chemical stability. The pharmaceutical composition according to the invention contains commonly used fillers and vehicles, e.g. lactose, mannitol, sorbitol, gliders, e.g. magnesium stearate, stearic acid and talc, furthermore substances facilitating disintegration, e.g. poly(vinylpoly-pyrrolidone), starch, cellulose and pigments, e.g. iron(III)-oxides, or organic dyes. The quantity of decomposition products in the compositions of the present invention is low even after a longer storage.

We have found that stable pharmaceutical compositions can be obtained by using beside paracetamol and drotaverine HCl commonly used saccharide type fillers and as stabilizer an organic acid. The above statement is true even in the case, when the outside or inside phase of the composition contains active ingredient e.g. codein phosphate too, and as glider magnesium stearate or other compound(s), affecting stability of the active ingredients used.

We have furthermore found, that by granulating the paracetamol and the drotaverine HCl active ingredients separately with the commonly used saccharide type fillers, using poly(vinyl-pyrrolidine) as vehicle, then homogenizing the two sorts of granules with disintegration facilitating substances and gliders, chemically stable oral composition can be obtained, which can be compressed to tablets or filled into capsules.
According to our experiences by compressing the two sorts of granules containing paracetamol and drotaverine HCl to layered tablets compositions with adequate stability can be obtained too.

According to a preferred embodiment of our invention as fillers untreated and pretreated starch and microcrystalline cellulose, as vehicle starch and poly(vinylpyrrolidone, as disintegration facilitating substance, poly(vinyl-poly-pyrrolidone), as gliders magnesium stearate and/or stearic acid, talc, as stabilizers pharmaceutically acceptable organic acids e.g. citric acid. maleic acid or tartaric acid, optionally ascorbic acid, can by used over the commonly used pigments e.g. iron(III)-oxides. The additional auxiliary materials which may be applied are listed in the Hungarian Pharmacopoeia or the European Pharmacopoeia.

The preferred embodiment of our invention is a composition containing paracetamol, drotaverine hydrochloride, optionally codein phosphate and starch, microcrystalline cellulose, magnesium stearate and a stabilizing agent in the form of tablets or granulates to be filled into capsules.

The supposed stabilizing mechanism of the organic acids used according to the invention might be interpreted as follows: in the presence of moisture the magnesium stearate and the saccharids facilitate by their proton-binding character the hydrolysis of the drotaverine hydrochloride salt, which hydrolysis is retarded by the organic acids as proton donors. The moisture can originate from the air and/or from the granulating liquid, furthermore from the auxiliary products.

As stabilizers such organic acids can be applied in compositions of the present invention, containing drotaverine HCl and paracetamol in combination, the order of acidic dissociation constants (K) of which is 10⁻³-10⁻¹², preferable less than 10⁻⁴, and at the same time they possess of antioxidant character.

A further subject of our invention is a process for the preparation of the above pharmaceutical compositions, according which paracetamol, drotaverine HCl and the organic acid applied as stabilizer are homogenized together with the filler(s), then the granulates to be filled into capsules and/or to be compressed to tablets are produced by wet granulation.
The tablets produced according to the invention are stable from physical and chemical aspects alike. They keep their stability for a long time under the conditions of accelerated and long term stability tests too.
We performed the following tests to compare the preservability of compisitions produced according to our invention containing stabilizer and compositions without stabilizer.
a) Impurity tests by spectrophotometry (p-aminophenol) and high performance liquid chromatography (decomposition products of drotaverine HCl):
   During the tests the quantitative change of the characteristic decomposition products of the preparations, namely that of p-aminophenol, the main decomposition product of paracetamol, and the decomposition products of drotaverine HCl were followed.
b) From the tests of pharmaceutical formulations first of all the disintegration of tablets has been studied according to the pharmacopeal requirements.
c) From the physical characteristics we examined on the first place the outside appearance of the tablets. The results are summed up in table I. From the data of the table it can be seen that chemical stability of the preparations containing citric acid or ascorbic acid is adequate.

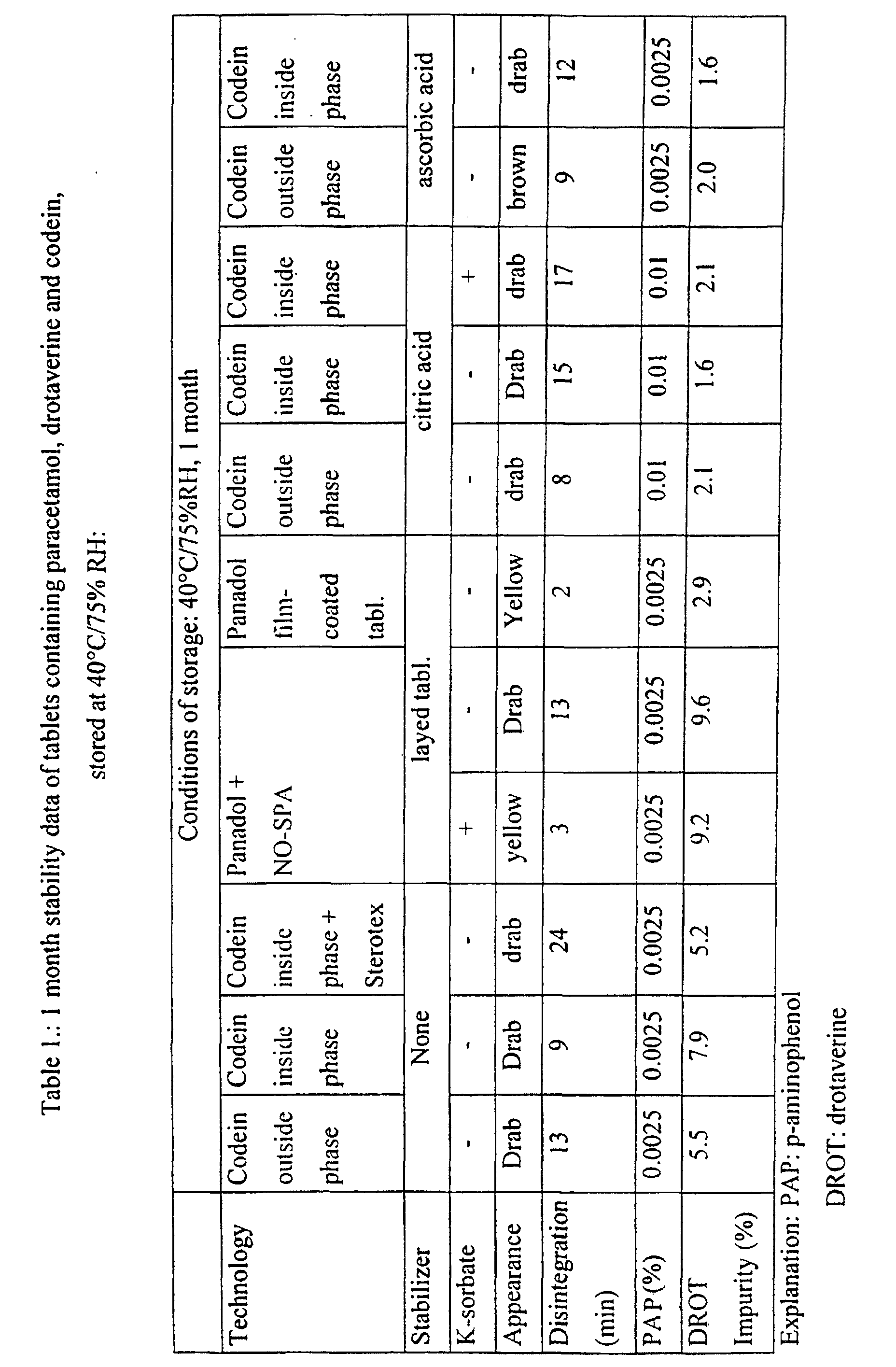

Further details of the invention are demonstrated by the examples, without limiting the scope of the invention to them. The active ingredients and auxiliary products and other additives used in the examples were of pharmacopeal quality, or suitable to pharmaceutical use.

### Example 1

5000 g paracetamol, 400 g drotaverine hydrochloride, 190 g maize starch, 500 g pretreated maize starch and 100 g ascorbic acid are homogenized for 5 minutes in a high speed homogenizer. 20 g poly(vinylpyrrolidone) (PVP K25) are dissolved in about 1300 ml of purified water. The homogenized powder mixture is granulated for about 5 minutes while adding slowly the PVP K25 solution. The wet granulate is dried (at about 30°C), until its moisture content reaches at least 2 %. 80 g codein phosphate, 150 g talc, 200 g microcrystalline cellulose and 130 g poly(vinylpyrrolidone) are added to the dry granulate, it is homogenized for about 5 minutes, then 30 g magnesium stearate is added and it is homogenized further for 5 minutes. The homogenized granulate is compressed.

### Example 2

5000 g paracetamol, 400 g drotaverine hydrochloride, 190 g maize starch, 480 g pretreated maize starch and 100 g ascorbic acid are homogenized for 5 minutes in a high speed homogenizer. 20 g poly(vinylpyrrolidone) (PVP K25) is dissolved in about 1300 ml of purified water. The homogenized powder mixture is granulated for about 5 minutes while adding slowly the PVP K25 solution. The wet granulate is dried (at about 30°C), until its moisture content reaches at least 2 %. 80 g codein phosphate, 150 g talc, 200 g microcrystalline cellulose and 130 g polyvinylpyrrolidone are added to the dry granulate, it is homogenized for about 5 to 10 minutes, then 50 g magnesium stearate is added to it and it is homogenized for further 5 minutes. The homogenized granulate is compressed.

### Example 3

5000 g paracetamol, 400 g drotaverine hydrochloride, 130 g maize starch, 390 g pretreated maize starch and 100 g ascorbic acid are homogenized for 5 minutes in a high speed homogenizer. 20 g poly(vinylpyrrolidone) (PVP K25) is dissolved in about 1300 ml of purified water. The homogenized powder mixture is granulated for about 5 minutes while adding slowly the PVP K25 solution. The wet granulate is dried (at about 30°C), until its moisture content reaches at least 2 %. 80 g codein phosphate, 150 g talc, 200 g microcrystalline cellulose and 130 g polyvinylpyrrolidone are added to the dry granulate, it is homogenized for about 5 minutes, then 200 g hydrogenated vegetable oil is added to it and it is homogenized for further 10 minutes. The homogenized granulate is compressed.

### Example 4

5000 g paracetamol, 400 g drotaverine hydrochloride, 160 g maize starch, 500 g pretreated maize starch and 100 g ascorbic acid are homogenized for 5 minutes in a high speed homogenizer. 20 g poly(vinylpyrrolidone) (PVP K25) is dissolved in about 1300 ml of purified water. The homogenized powder mixture is granulated for about 5 minutes while adding slowly the PVP K25 solution. The wet granulate is dried (at about 30°C), until its moisture content reaches at least 2 %. 80 g codein phosphate, 150 g talc, 200 g microcrystalline cellulose, 30 g yellow iron oxide and 130 g polyvinylpyrrolidone are added to the dry granulate, it is homogenized for about 5 minutes, then 30 g magnesium stearate is added to it, and it is homogenized for further 5 minutes. The homogenized granulate is compressed.

### Example 5

5000 g paracetamol, 400 g drotaverine hydrochloride, 214 g maize starch, 500 g pretreated maize starch and 100 g ascorbic acid are homogenized for 5 minutes in a high speed homogenizer. 20 g polyvinylpyrrolidone (PVP K25) is dissolved in about 1300 ml of purified water. The homogenized powder mixture is granulated for about 5 minutes while adding slowly the PVP K25 solution. The wet granulate is dried (at about 30°C), until its moisture content reaches at least 2 %. 80 g codein phosphate, 150 g talc, 200 g microcrystalline cellulose are added to the dry granulate, it is homogenized for 5 minutes, then 10 g magnesium stearate and 50 g stearic acid are added to it, and it is homogenized for further 4-5 minutes. The homogenized granulate is compressed.

### Example 6

5000 g paracetamol, 400 g drotaverine hydrochloride, 214 g maize starch, 500 g pretreated maize starch and 500 g ascorbic acid are homogenized for 5 minutes in a high speed homogenizer. 20 g polyvinylpyrrolidone (PVP K25) is dissolved in about 1300 ml of purified water. The homogenized powder mixture is granulated for about 5 minutes while adding slowly the PVP K25 solution. The wet granulate is dried (at about 30°C), until its moisture content reaches at least 2 %. 80 g codein phosphate, 150 g talc, 200 g microcrystalline cellulose are added to the dry granulate, it is homogenized for about 5 minutes, then 10 g magnesium stearate and 50 g stearic acid are added and it is homogenized further for 5 minutes. The homogenized granulate is compressed.

### Example 7

5000 g paracetamol, 400 g drotaverine hydrochloride, 217.5 g maize starch, 500 g pretreated maize starch and 100 g ascorbic acid are homogenized for 5 minutes in a high speed homogenizer. 20 g poly(vinylpyrrolidone) (PVP K25) and 2.5 g of Ariavit Sunset Yellow dye are dissolved in about 1300 ml of purified water. The homogenized powder mixture is granulated for about 5 minutes while adding slowly the PVP K25 solution. The wet granulate is dried (at about 30°C), until its moisture content reaches at least 2 %. 80 g codein phosphate, 150 g talc, 200 g microcrystalline cellulose and 130 g polyvinypyrrolidone are added to the dry granulate, it is homogenized for about 5 minutes, then 50 g stearic acid is added to it and it is homogenized for further 5 minutes. The homogenized granulate is compressed.

### Example 8

5000 g paracetamol, 400 g drotaverine hydrochloride, 214 g maize starch, 500 g pretreated maize starch, 100 g citric acid and 6 g potassium sorbate are homogenized for 5 minutes in a high speed homogenizer. 20 g poly(vinylpyrrolidone) (PVP K25) is dissolved in about 1300 ml of purified water. The homogenized powder mixture is granulated for about 5 minutes while adding slowly the PVP K25 solution. The wet granulate is dried (at about 30°C), until its moisture content reaches at least 2 %. 80 g codein phosphate, 150 g talc, 200 g microcrystalline cellulose are added to the dry granulate, it is homogenized for about 5 minutes, then 10 g magnesium stearate and 50 g stearic acid are added to it and it is homogenized for further 5 minutes. The homogenized granulate is compressed.

### Example 9

5000 g paracetamol, 400 g drotaverine hydrochloride. 80 g codein phosphate, 164 g maize starch, 4700 g pretreated maize starch, 100 g citric acid and 6 g potassium sorbate are homogenized for 5 minutes in a high speed homogenizer. 20 g poly(vinylpyrrolidone) (PVP K25) is dissolved in about 1300 ml of purified water. The homogenized powder mixture is granulated for about 5 minutes while adding slowly the PVP K25 solution. The wet granulate is dried (at about 30°C), until its moisture content reaches at least 2 %. 150 g talc, 200 g microcrystalline cellulose are added to the dry granulate, it is homogenized for about 5 minutes, then 50 g stearic acid is added to it and it is homogenized for further 5 minutes. The homogenized granulate is compressed.

## Claims

1. A stable, solid pharmaceutical composition comprising as active ingredients paracetamol and drotaverine hydrochloride in a mixture with one or more organic acid or potassium sorbate as stabilizers, one or more usually used pharmaceutical auxiliary materials and optionally additional active ingredient.

2. Composition according to claim 1.) containing codein phosphate as additional active ingredient.

3. Composition according to claims 1.) and 2.) containing as organic acid an organic acid. having a dissociation constant (K) of 10⁻³-10⁻¹².

4. Composition according to claim 3.) containing ascorbic acid or citric acid as the organic acid.

5. Composition according to claims 1.) and 2.) containing as filler and as disintegration facilitating vehicle starch and as glider substances stearic acid, the salts or esters of it or hydrogenated vegetable oils.

6. Composition according to claim 1.) containing paracetamol and drotaverine hydrochloride as active ingredients, ascorbic acid, citric acid or potassium sorbate as stabilizer, starch as filler and as disintegration facilitating vehicle, magnesium stearate, stearic acid, hydrogenated vegetable oil or talc as glider, iron(III)-oxide or Ariavit Sunset Yellow as colouring vehicle.

7. Compositon according to claims 1.) and 2.) containing paracetamol, drotaverine hydrochloride and codein phosphate as active ingredients, ascorbic acid, citric acid or potassium sorbate as stabilizer, starch as filler and as disintegration facilitating vehicle, magnesium stearate, stearic acid, hydrogenated vegetable oil or talc as glider, iron(III)-oxide or Ariavit Sunset Yellow as colouring vehicle and poly(vinylpyrrolidone).

8. Composition according to claim 1.) **characterised by** that it is in the form of granulates which can be filled into capsules or compressed to tablets.

9. Process for the preparation of a composition according to claim 1.) **characterised by** that wet granulation is used.

10. Process according to claim 9.) **characterised by** that paracetamol, drotaverine hydrochloride, starch, pretreated starch, the stabilizer organic acid and the polyvinylpyrrolidone are mixed in dry form, then the mixture is wet-granulated, dried, assorted, mixed with the glider and the disintegration facilitating substance, and then compressed to tablets.

## Patentansprüche

1. Stabile, feste pharmazeutische Zusammensetzung, die als Wirkstoffe Paracetamol und Drotaverinhydrochlorid in Mischung mit einer oder mehreren organischen Säuren oder Kaliumsorbat als Stabilisierungsmittel, einem oder mehreren üblicherweise verwendeten pharmazeutischen Hilfsstoffen und gegebenenfalls einem zusätzlichen Wirkstoff umfasst.

2. Zusammensetzung gemäß Anspruch 1.), die als zusätzlichen WirkstoffCodeinphosphat enthält.

3. Zusammensetzung gemäß den Ansprüchen 1.) und 2.), die als organische Säure eine organische Säure mit einer Dissoziationskonstante (K) von 10⁻³-10⁻¹² enthält.

4. Zusammensetzung gemäß Anspruch 3.), die als organische Säure Ascorbinsäure oder Zitronensäure enthält.

5. Zusammensetzung gemäß den Ansprüchen 1.) und 2.), die als Füllmittel und auflösungsförderndes Vehikel Stärke und als Gleitsubstanzen Stearinsäure, deren Salze oder Ester oder gehärtete Pflanzenöle enthält.

6. Zusammensetzung gemäß Anspruch 1.), die als Wirkstoffe Paracetamol und Drotaverinhydrochlorid, als Stabilisierungsmittel Ascorbinsäure, Zitronensäure oder Kaliumsorbat, als Füllmittel und auflösungsförderndes Vehikel Stärke, als Gleitstoff Magnesiumstearat, Stearinsäure, gehärtetes Pflanzenöl oder Talk, als Farbvehikel Eisen(III)-Oxid oder Ariavit Sunset Yellow enthält.

7. Zusammensetzung gemäß den Ansprüchen 1.) und 2.), die als Wirkstoffe Paracetamol, Drotaverinhydrochlorid und Codeinphosphat, als Stabilisierungsmittel Ascorbinsäure, Zitronensäure oder Kaliumsorbat, als Füllmittel und auflösungsförderndes Vehikel Stärke, als Gleitstoff Magnesiumstearat, Stearinsäure, gehärtetes Pflanzenöl oder Talk, als Farbvehikel Eisen(III)-Oxid oder Ariavit Sunset Yellow und Poly(vinylpyrrolidon) enthält.

8. Zusammensetzung gemäß Anspruch 1.), **dadurch gekennzeichnet, dass** sie in Form von Granulaten vorliegt, welche in Kapseln gefüllt oder zu Tabletten gepresst werden können.

9. Verfahren zur Herstellung einer Zusammensetzung gemäß Anspruch 1.), **dadurch gekennzeichnet, dass** eine Feuchtgranulierung angewandt wird.

10. Verfahren gemäß Anspruch 9.), **dadurch gekennzeichnet, dass** Paracetamol, Drotaverinhydrochlorid, Stärke, vorbehandelte Stärke, die organische Säure als Stabilisierungsmittel und das Polyvinylpyrrolidon in trockener Form vermischt werden, die Mischung daraufhin feucht granuliert, getrocknet, gesichtet, mit dem Gleitstoff und der auflösungsfördernden Substanz vermischt und danach zu Tabletten gepresst wird.

## Revendications

1. Composition pharmaceutique solide stable comprenant en tant que principes actifs du paracétamol et du chlorhydrate de drotavérine en mélange avec un acide organique ou plus ou du sorbate de potassium en tant que stabilisants, un ou plusieurs matériaux auxiliaires pharmaceutiques habituellement utilisés et facultativement un principe actif supplémentaire.

2. Composition selon la revendication 1, contenant du phosphate de codéine en tant que principe actif supplémentaire.

3. Composition selon les revendications 1 et 2, contenant en tant qu'acide organique un acide organique présentant une constante de dissociation (K) de 10⁻³ à 10⁻¹².

4. Composition selon la revendication 3, contenant de l'acide ascorbique ou de l'acide citrique en tant qu'acide organique.

5. Composition selon les revendications 1 et 2, contenant en tant que charge et en tant que véhicule facilitant le délitage, de l'amidon, et tant que substances de glissement, de l'acide stéarique, les sels ou les esters de celui-ci ou des huiles végétales hydrogénées.

6. Composition selon la revendication 1, contenant du paracétamol et du chlorhydrate de drotavérine en tant que principes actifs, de l'acide ascorbique, de l'acide citrique ou du sorbate de potassium en tant que stabilisant, de l'amidon en tant que charge et en tant que véhicule facilitant le délitage, du stéarate de magnésium, de l'acide stéarique, une huile végétale hydrogénée ou du talc en tant qu'agent de glissement, de l'oxyde de fer (III) ou du jaune Ariavit Sunset Yellow en tant que véhicule de coloration.

7. Composition selon les revendications 1 et 2, contenant du paracétamol, du chlorhydrate de drotavérine et du phosphate de codéine en tant que principes actifs, de l'acide ascorbique, de l'acide citrique ou du sorbate de potassium en tant stabilisant, de l'amidon en tant que charge et en tant que véhicule facilitant le délitage du stéarate de magnésium, de l'acide stéarique, une huile végétale hydrogénée ou du talc en tant qu'agent de glissement, de l'oxyde de fer (III) ou du jaune Ariavit Sunset Yellow en tant que véhicule colorant et de la poly(vinylpyrrolidone).

8. Composition selon la revendication 1, **caractérisée par le fait qu'**elle est sous forme de granulés qui peuvent être introduits dans des capsules ou compressé en comprimés.

9. Procédé de préparation d'une composition selon la revendication 1, **caractérisé par le fait qu'**une granulation humide est utilisée.

10. Procédé selon la revendication 9, **caractérisé par le fait que** du paracétamol, du chlorhydrate de drotavérine, de l'amidon, de l'amidon pré-traité, l'acide organique stabilisant et la polyvinylpyrrolidone sont mélangés sous forme sèche, puis le mélange est granulé à l'état humide, séché, classé, mélangé avec l'agent de glissement et la substance facilitant le délitage, et ensuite compressé en comprimés.
